# EUROPEAN PATENT SPECIFICATION

(11) **EP 1 892 295 B1**
(45) Date of publication and mention of the grant of the patent: **25.07.2012**
(21) Application number: 07114679.9
(22) Date of filing: 21.08.2007
(51) Int. Cl.: C12Q 1/68, A61B 10/00, C12N 1/02, C12N 15/10, B01L 7/00

(54) **Method and device of isolating and amplifying nucleic acid from microorganism cell using nonplanar solid substrate**
Verfahren und Vorrichtung zur Isolierung und Verstärkung von Nukleinsäure aus Zellen von Mikroorganismen mittels eines nonplanaren festen Substrats
Procédé et dispositif d'isolation et d'amplification d'acide nucléique d'une cellule de microorganisme utilisant un substrat solide non planaire

(30) Priority: 21.08.2006 KR 20060079056; 21.08.2006 KR 20060079053; 21.08.2006 KR 20060079054; 21.08.2006 KR 20060079055
(43) Date of publication of application: 27.02.2008
(73) Proprietor: Samsung Electronics Co., Ltd., Suwon-si, Gyeonggi-do (KR)
(72) Inventor: Hwang, Kyu-youn, c/o Samsung Adv. Ins. of Tech., Gyeonggi-do (KR); Jeong, Sung-young, c/o Samsung Adv. Ins. of Tech., Gyeonggi-do (KR); Kim, Joon-ho, c/o Samsung Adv. Ins. of Tech., Gyeonggi-do (KR); Han, Jung-im, c/o Samsung Adv. Ins. of Tech., Gyeonggi-do (KR)
(74) Representative: Grünecker, Kinkeldey, Stockmair & Schwanhäusser

(56) References cited:
- WO-A-98/51693
- WO-A-03/010278
- WO-A-2004/087226
- WO-A-2005/093065
- ACARTURK T O ET AL: "Control of attachment, morphology, and proliferation of skeletal myoblasts on silanized glass." JOURNAL OF BIOMEDICAL MATERIALS RESEARCH 15 MAR 1999, vol. 44, no. 4, 15 March 1999 (1999-03-15), pages 355-370, XP002468347 ISSN: 0021-9304
- SPARGO ET AL: "Spatially controlled adhesion, spreading, and differentiation of endothelial cells on self-assembled molecular monolayers" PROCEEDINGS OF THE NATIONAL ACADEMY OF SCIENCES OF USA, NATIONAL ACADEMY OF SCIENCE, WASHINGTON, DC, US, vol. 91, November 1994 (1994-11), pages 11070-11074, XP002103614 ISSN: 0027-8424
- LIU Q Y ET AL: "Synaptic connectivity in hippocampal neuronal networks cultured on micropatterned surfaces." BRAIN RESEARCH. DEVELOPMENTAL BRAIN RESEARCH 14 APR 2000, vol. 120, no. 2, 14 April 2000 (2000-04-14), pages 223-231, XP002468348 ISSN: 0165-3806
- YANG CHANGMING ET AL: "Electrically driven microseparation methods for pesticides and metabolites: III. Capillary electrochromatography with novel silica-based stationary phases having a surface-bound surfactant moiety" ELECTROPHORESIS, vol. 21, no. 10, June 2000 (2000-06), pages 1977-1984, XP002468349 ISSN: 0173-0835

## Description

### BACKGROUND OF THE INVENTION

### 1. Field of the Invention

The present invention relates to methods of lysing isolated cell and of isolating a nucleic acid from a microorganism cell using a nonplanar solid substrate, a method of amplifying a nucleic acid using the isolated nucleic acid as a template, and a device including the nonplanar solid substrate for isolating and amplifying a nucleic acid.

### 2. Description of the Related Art

There are several conventional methods of purifying a nucleic acid using a solid phase. For example, US Patent No. 5,234,809 discloses a method of purifying a nucleic acid using a solid phase to which the nucleic acid is bound. This method, however, is time-consuming and complex, and thus, is unsuitable for a lab-on-a-chip (LOC). In addition, a chaotropic substance must be used in order to perform this method. That is, without using a chaotropic substance, a nucleic acid is not bound to the solid phase.

US Patent No. 6,291,166 discloses a method of archiving a nucleic acid using a solid phase matrix. According to this method, the nucleic acid is irreversibly bound to the solid phase matrix. Such irreversible binding enables delayed analysis or repeated analysis after a nucleic acid-solid phase matrix composite is stored. However, in this method, a substance having a positively charged surface, such as alumina, is activated by a base substance, such as NaOH, and then a nucleic acid is irreversibly bound to the activated alumina. As a result, the bound nucleic acid cannot be isolated from the alumina.

US Patent No. 5,705,628 discloses a method of reversibly and non-specifically binding DNA of a DNA-containing solution, which has been mixed with a salt and polyethylene glycol, to a magnetic microparticle having a carboxyl group-coated surface. This method uses a magnetic microparticle having a carboxyl group-coated surface, a salt, and polyethylene glycol, in order to isolate DNA.

WO 03/010278 A relates to an entirely automated process and device for detecting the presence of pathogenic organisms in a water sample. The process comprises the steps: concentration of the pathogenic organism, breaking open the cells, concentration of DNA and/or of RNA, at least one nucleic acid amplification and qualitatively and/or quantitatively detecting DNA and/or RNA representative of the water contamination by said pathogenic organism. The device accordingly comprises units for performing the method steps as well as pumping means, automatic sampling and distribution means and at least one control logic system which controls fully automatically the sequential treatment steps.

WO 2005/093065 A discloses a method for the isolation of nucleic acid from a sample containing cells or complex biological material. The method comprises the steps (i) mixing said sample with a suspension comprising a buffer and a solid support, said suspension having a pH value below pH 5 characterized in that said buffer causes lysis of said cells or said biological material, wherein subsequent to lysis, the nucleic acid released is bound to said solid support: (ii) washing the generated nucleic acid/solid support complex: and (iii) diluting the nucleic acid from the solid support.

WO 98/51693 A refers to a kit for and a method of isolating nucleic acids from a sample of cells, comprising: binding cells and said cell sample to a solid support-to isolate cells from the sample: lysing the isolated cells; and binding nucleic acid released from said lysed cells to said solid support.

As described above, conventional methods of isolating and purifying a nucleic acid require the addition of a high-concentration reagent for DNA binding. However, such addition can affect a subsequent process, such as a polymerase chain reaction (PCR), and thus, said methods cannot be performed on a lab-on-a-chip (LOC). In addition, in the prior art, conventional methods of isolating and purifying a nucleic acid are used independently from methods of purifying or concentrating a cell or virus. A method combining purifying or concentrating a cell or virus with isolating a nucleic acid from the resulting purified or concentrated cell or virus is not known.

Accordingly, there is a need to develop a method in which a microorganism cell is isolated or concentrated by binding the microorganism cell to a solid surface, such as a substrate, and straight forward, a nucleic acid derived from the microorganism cell is purified and concentrated.

### SUMMARY OF THE INVENTION

The present invention provides methods of lysing isolated cells and of isolating a cell or virus using a nonplanar solid substrate and isolating a nucleic acid from the isolated cell or virus.

The present invention also provides a method of amplifying the isolated nucleic acid.

The present invention also provides a device including a nonplanar solid substrate for isolating and amplifying a nucleic acid.

### BRIEF DESCRIPTION OF THE DRAWINGS

The above and other features and advantages of the present invention will become more apparent by describing in detail exemplary embodiments thereof with reference to the attached drawings in which:
FIG. 1 is a graph of fluorescence intensity with respect to the concentration of DNAs in a NaOH solution of pH 12.0 and in a phosphate buffer of pH 7.0;
FIG. 2 is a graph of the DNA elution rate with respect to the pH of a DNA sample after the DNA sample is loaded onto a fluidic device with a pillar array;
FIG. 3 is a graph of the fluorescence intensity, corresponding to the concentration of DNA present in a cell lysate obtained by cell lysis, with respect to the cell concentration of a sample;
FIG. 4 is a graph illustrating the DNA elution efficiency measured in relation to the method by the cells bound to the surface of a nonplanar solid substrate are lysed;
FIG. 5 is the picture of an electrophoresis gel showing the concentration of amplified DNA obtained by real time PCR amplification of Table 1;
FIG. 6 is a graph illustrating the results of real time PCR amplification after an *E*.*coli*-containing urine sample is loaded onto a chamber having a pillar array of a fluidic device and undergoes cell lysis and cell washing;
FIG. 7 is a graph illustrating the results of real time PCR amplification after an *E.coli*-containing whole blood sample is loaded onto a chamber having a pillar array of a fluidic device and undergoes cell lysis and DNA isolation; and
FIG. 8 is the picture of an electrophoresis gel showing the results of electrophoresis after an *E.coli*-containing whole blood sample is loaded onto a chamber having a pillar array of a fluidic device and undergoes cell lysis, DNA extraction, real time PCR amplification, and electrophoresis.

### DETAILED DESCRIPTION OF THE INVENTION

The present invention will now be described in more detail with reference to the accompanying drawings, in which exemplary embodiments of the invention are shown.

A method of isolating a nucleic acid from a cell according to an embodiment of the present invention includes: contacting a nonplanar solid substrate with a cell-containing sample in a liquid medium having a pH of 3.0 to 6.0, whereby the cells are bound to the nonplanar solid substrate; and lysing the cells bound to the nonplanar solid substrate, and wherein the nonplanar solid substrate has a hydrophobic surface having a water contact angle of 70° to 95°.

The cells bind to the nonplanar solid substrate by the contacting.

In a system comprising a liquid medium that is in contact with a solid substrate, a cell, preferably a microorganism, such as bacteria, fungi or a virus, can be present in the liquid medium or can be bound to the solid substrate. Whether the microorganism exists in the liquid medium or is bound to the solid substrate is determined by the difference in surface tensions of the liquid medium and the microorganism. For example, when the liquid medium has a greater surface tension than the microorganism, the microorganism may be easily bound to a solid substrate having low surface tension, that is, to a hydrophobic solid substrate; when the liquid medium has a smaller surface tension than the microorganism, the microorganism may be easily bound to a solid substrate having high surface tension, that is, to a hydrophilic solid substrate; and when the liquid medium and microorganism have nearly the same surface tension, the surface tension does not affect the binding of microorganism to the solid substrate and other interaction factors, such as electrostatic interaction, may affect such binding (see Applied and Environmental Microbiology, July 1983, p.90-97). In addition, it is known that microorganisms can be bound to a solid substrate by electrostatic attraction as well as by a thermodynamic approach based on the surface tension. However, such bindings occur very slowly and its bound quantity was very small.

The inventors of the present invention made efforts to address these problems and found that a large amount of microorganism cells could be isolated by contacting a nonplanar solid substrate with a microorganism cell-containing sample in a liquid medium having a pH of 3.0 to 6.0. The large amount of isolated cells may be obtained because the increased surface area of a nonplanar solid substrateis. Further, by using a liquid medium having a pH 3.0 to 6.0, the cell membrane of a cell, such as a microorganism cell is denatured, and thus, the solubility of the microorganism cells in the liquid medium is lowered so that more microorganism cells tend to bind to the solid surface. However, the present invention is not limited to such a technique.

During the contacting process, the sample can be any sample containing a cell, preferably a microorganism cell. For example, the sample can be a biological sample containing a microorganism cell, a clinical sample containing a microorganism cell, or a lab sample containing a microorganism cell. In the present specification, a biological sample refers to a sample that includes or is formed of a cell or tissue, such as a cell or biological liquid isolated from an individual. The individual can be an animal including a human. The biological sample can be saliva, sputum, blood, blood cells (for example, red blood cells or white blood cells), amniotic fluid, serum, semen, bone marrow, tissue or a micro needle biopsy sample, urine, peritoneum fluid, pleura fluid, or cell cultures. In addition, the biological sample can be a tissue section, such as a frozen section taken for a histological object. Preferably, the biological sample is a clinical sample obtained from a human patient. More preferably, the biological sample is blood, urine, saliva, or sputum.

According to an embodiment of the present invention a microorganism cell that is to be isolated can be a bacterial cell, fungus, or a virus.

During the contacting process, the biological sample can be diluted in the liquid medium that may preferably be a solution or buffer that may buffer the microorganism cell in an environment having a low pH. The buffer can be a phosphate buffer, such as sodium phosphate of pH 3.0 to 6.0, or an acetate buffer, such as sodium acetate of pH 3.0 to 6.0. The degree of dilution is not limited, and the biological sample can be diluted in the liquid medium in a range of 1:1 to 1:1,000, and preferably, 1:1 to 1:10.

During the contacting process, the liquid medium may have a salt concentration of 10 mM to 500 mM, and preferably, 50 mM to 300 mM. That is, the sample is contacting the nonplanar solid substrate in a liquid medium having an acetate or phosphate ion concentration of 10 mM to 500 mM, preferably 50 mM to 300 mM. The above described preferred pH-range and salt concentration refers to the pH and concentration of the liquid medium containing the cell-containing sample present in the contacting step.

During the contacting process, the solid substrate has a nonplanar shape so that the surface area of the nonplanar solid substrate can be increased compared to a planar surface. For example, the nonplanar solid substrate may have a corrugated surface. In the present specification, a corrugated surface refers to a non-level surface having grooves and ridges. The corrugated surface can be a surface having a plurality of pillars or a sieve-shaped surface having a plurality of pores. However, the corrugated surface may have other shapes.

During the contacting process, the nonplanar solid substrate may have various shapes. For example, the nonplanar solid substrate can be a solid substrate having a surface with a plurality of pillars, a bead-shaped solid substrate, or a sieve-shaped solid substrate having a plurality of pores in its surface. The solid substrate can be a single solid substrate or a combination of solid substrates, such as a solid substrate assembly that fills a tube or container.

During the contacting process, the nonplanar solid substrate may form an inner wall of a microchannel or microchamber of a microfluidic device. Accordingly, the method of isolating a nucleic acid from a microorganism cell according to the current embodiment of the present invention can be used in a fluidic device or microfluidic device having at least one inlet and outlet connected through a channel or microchannel.

As used herein, the term "microfluidic device" incorporates the concept of a microfluidic device that comprises microfluidic elements such as, e.g., microfluidic channels (also called microchannels or microscale channels). As used herein, the term "microfluidic" refers to a device component, e.g., chamber, channel, reservoir, or the like, that includes at least one cross-sectional dimension, such as depth, width, length, diameter, etc. of from about 0.1 micrometer to about 1000 micrometer. Thus, the term "microchamber" and "microchannel" refer to a channel and a chamber that includes at least one cross-sectional dimension, such as depth, width, and diameter of from about 0.1 micrometer to about 1000 micrometer, respectively.

In the method of isolating a nucleic acid from a microorganism cell according to the current embodiment of the present invention, during the contacting process, the nonplanar solid substrate may have a surface having a plurality of pillars. A method of forming pillars on a solid substrate is well known in the art. For example, micro pillars can be formed in a high density structure using a photolithography process used in a semiconductor manufacturing process.

The pillars may have an aspect ratio, the height of the pillar : the length of the cross section of 1:1-20:1, but the aspect ratio is not limited thereto. The term "aspect ratio" used herein refers to a ratio of a height of a pillar to the length of a cross section of a pillar. The length of a cross section of the pillar refers to a diameter when the shape of the cross section is a circle and it refers to an average value of the length of each side when the shape of the cross section is a rectangle.

In the pillar structure, the ratio of the height of the pillars to the distance between adjacent pillars may be in the range of 1:1 to 25:1. The distance between adjacent pillars may be in the range of 5 µm to 100 µm.

In the method of isolating a nucleic acid from a microorganism cell according to the present invention, during the contacting process, the nonplanar solid substrate may be hydrophobic and have a water contact angle of 70° to 95°. The hydrophobic property of the nonplanar solid substrate having a water contact angle of 70° to 95° can be obtained by coating octadecyldimethyl(3-trimethoxysilyl propyl)ammonium (TMSAC), tridecafluorotetrahydrooctyltrimethoxysilane (FTEOS) or CF₃(CF₂)₃CH₂CH₂SI(OCH₃)₃, CF₃(CF₂)₅CH₂CH₂SI(OCH₃)₃, CF₃(CF₂)₇CH₂CH₂SI(OCH₃)₃, CF₃ (CF₂)₉CH₂CH₂SI(OCH₃)₃, (CF₃)₂CF(CF₂)₄CH₂CH₂SI(OCH₃)₃, (CF₃) ₂CF(CF₂)₆CH₂CH₂SI(OCH₃)₃, (CF₃)₂CF(CF₂)₈CH₂CH₂SI(OCH₃)₃, CF₃ (C₆H₄)C₂H₄Si(OCH₃)₃, CF₃ (CF₂)₃ (C₆H₄)C₂H₄Si(OCH₃)₃, CF₃ (CF₂)₅(C₆H₄)C₂H₄Si(OCH₃)₃, CF₃(CF₂)₇(C₆H₄)C₂H₄Si(OCH₃)₃, CF₃(CF₂) ₃CH₂CH₂SiCH₃(OCH₃)₂, CF₃(CF₂)₅CH₂CH₂SiCH₃(OCH₃)₂, CF₃ (CF₂)₇CH₂CH₂SiCH₃(OCH₃)₂, CF₃(CF₂)₉CH₂CH₂SiCH₃(OCH₃)₂, (CF₃) ₂CF(CF₂)₄CH₂CH₂SiCH₃ (OCH₃)₂, (CF₃)₂CF(CF₂)₆CH₂CH₂SiCH₃ (OCH₃)₂, (CF₃) ₂CF(CF₂)₈CH₂CH₂SiCH₃ (OCH₃) ₂, CF₃ (C₆H₄)C₂H₄SiCH₃ (OCH₃) ₂, CF₃ (CF₂) ₃ (C₆H₄)C₂H₄SiCH₃(OCH₃)₂, CF₃(CF₂)₅(C₆H₄)C₂H₄SiCH₃(OCH₃)₂, CF₃ (CF₂)₇(C₆H₄)C₂H₄SiCH₃(OCH₃)₂, CF₃(CF₂)₃CH₂CH₂Si(OCH₂CH₃)₃, CF₃ (CF₂)₅CH₂CH₂Si(OCH₂CH₃)₃, or CF₃ (CF₂)₇CH₂CH₂Si(OCH₂CH₃) onto the surface or defined areas of a surface of the solid substrate. For example, a SiO₂ layer of a solid support may be coated with a self-assembled monolayer (SAM) of a compound selected from the group consisting of TMSAC and FTEOS to provide a water contact angle of 70-95°.

In this application, the term "water contact angle" refers to water contact angle measured by a Kruss Drop Shape Analysis System type DSA 10 Mk2. A droplet of 4 µl deionized water is automatically placed on the sample. The droplet was monitored every 0.2 seconds for a period of 10 seconds by a CCD-camera and analyzed by Drop Shape Analysis software (DSA version 1.7, Kruss). The complete profile of the droplet was fitted by the tangent method to a general conic section equation. The angles were determined both at the right and left side. An average value is calculated for each drop and a total of five drops per sample are measured. The average of the five drops is taken the contact angle.

In the method of isolating a nucleic acid from a microorganism cell according to the the present invention, during the contacting process, the nonplanar solid substrate may have at least one amine-based functional group at its surface. The surface with the amine-based functional group may be obtained by coating a compound selected from the group consisting of polyethyleneiminetrimethoxysilane (PEIM), aminopropyltriethoxysilane, N-(3-trimethoxysily)-propyl)ethylenediamine, and N-trimethoxysilylpropy-N,N,N-chloride trimethylammonium onto the solid substrate or defined regions thereof. For example, the coated surface can be obtained by self-assembled molecule (SAM) coating polyethyleneiminetrimethoxysilane (PEIM) on a SiO₂ layer of the solid substrate. The amine-based functional group is positively charged at a pH of 3.0-6.0. The surface with the amine-based functional group can more efficiently attach a microorganism cell which is present in a human body fulid such whole blood, saliva, urine etc., especially urine.

In the method of isolating a nucleic acid from a microorganism cell according to the current embodiment of the present invention, during the contacting process, the nonplanar solid substrate can be a substrate formed of any kind of material that has the water contact angle in the range described above and has at least one amine-based functional group at its surface. For example, the nonplanar solid substrate can be formed of glass, silicon wafer, plastic, or the like, but is not limited thereto. When a nonplanar solid substrate with a surface having a water contact angle of 70° to 95° and a surface having at least one amine-based functional group is contacted with a sample containing a microorganism cell in the liquid medium, the microorganism cell is assumed to be bound to the nonplanar solid substrate. However, the present invention is not limited to such a specific mechanism.

The method of isolating a nucleic acid from a microorganism cell according to the current embodiment of the present invention may further include, after the contacting process, washing the nonplanar solid substrate for removing unbound matters, excluding the target microorganism cell bound to the substrate, which are not bound to the nonplanar solid substrate. During the washing or removal process, any solution that does not release the target microorganism cell bound to the nonplanar solid substrate from the nonplanar solid substrate and removes impurities that may adversely affect subsequent processes can be used. For example, an acetate buffer or phosphate buffer which is used as a binding buffer can be used as the washing solution. The washing solution may have a pH of 3.0 to 6.0.

In the present specification, "isolation of a cell or a nucleic acid" intends to mean to include concentrating the cell or nucleic acid in the sample as well as purely separating the cell or nucleic acid, respectively.

The method of isolating a nucleic acid from the microorganism cell according to the current embodiment of the present invention includes lysing the cells bound to the nonplanar solid substrate.

The lysing of the microorganism cells can be performed using any lysing method known in the art. The lysis method can be boiling lysis, laser lysis, lysis using a chemical compound, or electrochemical lysis, such as electrolysis, but is not limited thereto.

In the method of isolating a nucleic acid from a microorganism cell according to the current embodiment of the present invention, the microorganism cell may be lysed in any liquid medium having a pH of 3.0 to 6.0 to bind a nucleic acid derived from the cell lysate to the nonplanar solid substrate. The lysis can be performed in a phosphate buffer or an acetate buffer having the respective pH.

In the current embodiment, the nucleic acid derived from the lysed microorganism cell is bound to the nonplanar solid substrate. Removing cell debris, and the like matters, which are not bound to the nonplanar solid substrate, can isolate the bound nucleic acid.

Accordingly, the method according to the current embodiment may further include, after the lysing process, washing the nonplanar solid substrate to remove unbound matters, which are not bound to the nonplanar solid substrate.

During the washing process, the washing solution can be any solution which does not release the bound nucleic acid from the nonplanar solid substrate and removes materials which are not bound to the nonplanar solid substrate. More specifically, the washing solution may be a solution having these properties described above and which removes impurities that may adversely affect subsequent processes. The washing solution can be an acetate buffer or phosphate buffer which can also be used as the binding buffer used when contacting the cell-containing sample with the nonplanar solid substrate. The washing solution may be a buffer having a pH of 3.0 to 6.0.

In the current embodiment, the nucleic acid bound to the nonplanar solid substrate can be used itself, or the bound nucleic acid can be extracted from the nonplanar solid substrate.

Accordingly, the method according to the current embodiment may further include extracting or eluting the nucleic acid bound to the nonplanar solid substrate.

During the extracting or elution process, the extracting solution may be any solution known in the art which can release the nucleic acid bound to the nonplanar solid substrate from the nonplanar solid substrate. For example, the extracting solution can be a solution having a high pH. By using a solution having a high pH, hydroxide ions (OH-) make the surface of the nonplanar solid substrate anionic so that anionic DNA is expelled and released from the negatively charged nonplanar solid substrate. The extracting solution can be a solution of pH 11 or more, such as a NaOH solution.

In a method of isolating a nucleic acid from a microorganism cell according to another embodiment of the present invention, the microorganism cell may be lysed in a solution of pH 11 to 14. Such lysis in a solution having a high pH can minimize the binding of DNA to a nonplanar solid substrate so that DNA of the lysed cells remains in the liquid medium and can be easily extracted. By using a solution of high pH, hydroxide ions (OH-) make the surface of the nonplanar solid substrate anionic so that anionic DNA of the cell lysate after the lysis is not bound to the nonplanar solid substrate but rather remains in the liquid. That is, if the object is to bind a microorganism cell to a nonplanar solid substrate and to extract DNA derived from the microorganism cell, it is desired that the microorganism cell is lysed in a solution having a high pH and the extraction is performed using the same solution having a high pH.

Therefore, the method of isolating a nucleic acid from a microorganism cell according to the current embodiment may further include purifying the nucleic acid from a lysate obtained during the lysing process. The purifying method can be any method known in the art.

The present invention also provides a method of amplifying a nucleic acid using as a template the nucleic acid that is isolated using the method of isolating a nucleic acid from a cell according to an embodiment of the present invention.

The present invention also provides a method of amplifying a nucleic acid using as a template a nucleic acid directly present in the cell lysate, said method comprising the step of contacting a nonplanar solid substrate with a cell-containing sample in a liquid medium having a pH of 3.0 to 6.0 wherein the nonplanar solid substrate has a hydrophobic surface having a water contact angle of 70° to 95°; and binding cells to the nonplanar solid substrate; and lysing the cells bound to the nonplanar solid substrate, wherein the cell is a microorganism cell, and amplifying the target nucleic acid using a nucleic acid directly present in the cell lysate as a template.

The amplifying of a nucleic acid can be performed using any amplifying method known in the art, for example, using PCR. The method of isolating a nucleic acid from a microorganism cell is described above.

In the method of amplifying a nucleic acid according to the current embodiment of the present invention, the nucleic acid can be DNA or RNA, and preferably DNA.

In the method of amplifying a nucleic acid according to the current embodiment of the present invention, the nucleic acid can be isolated and amplified in the same container including the nonplanar solid substrate. The container can be a microchannel, a microchamber, or a tube, but is not limited thereto. For example, the container may be a microchamber of a microfluidic device which is equipped with a PCR device. The PCR device includes a heater and a cooler. Therefore, in the method of amplifying a nucleic acid according to the current embodiment of the present invention, a nucleic acid is extracted in a microchamber and the extracted nucleic acid is amplified in the same microchamber.

In the method of amplifying a nucleic acid according to the current embodiment of the present invention, the isolation and amplification of the nucleic acid can be performed in different containers. For example, the isolation of the nucleic acid can be performed in a first container including the nonplanar solid substrate, and the amplification of the nucleic acid can be performed in a different second container that may or may not be in fluid communication with the first container including the nonplanar solid substrate. The second container can be a microchannel, a microchamber, or a tube, but is not limited thereto. For example, the isolation of the nucleic acid can be performed in a first microchannel or first microchamber comprising a nonplanar solid substrate of a microfluidic device, and the isolated nucleic acid is then transported to a second microchannel or second microchamber in which the amplification is performed.

A device for isolating and amplifying a nucleic acid according to an embodiment of the present invention includes: a reaction chamber including a nonplanar solid substrate; a heating unit capable of heating the reaction chamber; and a temperature controlling unit capable of controlling the heating unit wherein the nonplanar solid substrate has a hydrophobic surface having a water contact angle of 70° to 95°C.

In the device for isolating and amplifying a nucleic acid according to the current embodiment of the present invention, the solid substrate has a nonplanar surface so that it has greater surface area than a planar solid substrate. The nonplanar solid substrate may have a corrugated surface. In the present specification, the corrugated surface refers to a non-level surface having grooves and ridges. The corrugated surface can be a surface with a plurality of pillars or a sieve-shaped surface with a plurality of pores. However, the corrugated surface may have other shapes.

In the device for isolating and amplifying a nucleic acid according to the current embodiment of the present invention, the nonplanar solid substrate may have various shapes. For example, the nonplanar solid substrate can be selected from a solid substrate having a surface with a plurality of pillars, a bead-shaped solid substrate, and a sieve-shaped solid substrate having a plurality of pores in its surface. The solid substrate can be a single solid substrate or a combination of solid substrates, such as a solid substrate assembly which fills a tube or container.

In the device for isolating and amplifying a nucleic acid according to the current embodiment of the present invention, the nonplanar solid substrate may form an inner wall of a microchannel or microchamber of a microfluidic device. Accordingly, the device for isolating and amplifying a nucleic can be a fluidic device or microfluidic device having at least one inlet and outlet connected through a channel or microchannel. That is, the device for isolating and amplifying a nucleic acid according to the current embodiment of the present invention can be a fluidic device or microfluidic device in which the isolation and PCR of a nucleic acid can be performed in the same chamber.

In a device for isolating and amplifying a nucleic acid according to the current embodiment of the present invention, the nonplanar solid substrate may have a surface having a plurality of pillars. A method of forming pillars on a solid substrate is well known in the art. For example, micro pillars can be formed in a high density structure using a photolithography process used in a semiconductor manufacturing process.

The pillars may have an aspect ratio, the height of the pillar : the length of the cross section of 1:1-20:1, but the aspect ratio is not limited thereto. The term "aspect ratio" used herein refers to a ratio of a height of a pillar to the length of a cross section of a pillar. The length of a cross section of the pillar refers to a diameter when the shape of the cross section is a circle and it refers to an average value of the length of each side when the shape of the cross section is a rectangle.

In the pillar structure, the ratio of the height of the pillars to the distance between adjacent pillars may be in the range of 1:1 to 25:1. The distance between adjacent pillars may be in the range of 5 µm to 100 µm.

In the device for isolating and amplifying a nucleic acid according to the present invention, the nonplanar solid substrate may be hydrophobic, having a water contact angle of 70° to 95°. The hydrophobic property of the nonplanar solid substrate having a water contact angle of 70° to 95° can be obtained by coating octadecyldimethyl(3-trimethoxysilyl propyl)ammonium (TMSAC), tridecafluorotetrahydrooctyltrimethoxysilane (FTEOS) or CF₃(CF₂)₃CH₂CH₂SI(OCH₃)₃, CF₃ (CF₂)₅CH₂CH₂SI(OCH₃)₃, CF₃ (CF₂)₇CH₂CH₂SI(OCH₃)₃, CF₃ (CF₂)₉CH₂CH₂SI(OCH₃)₃, (CF₃)₂CF(CF₂)₄CH₂CH₂SI(OCH₃)₃, (CF₃)₂CF(CF₂)₆CH₂CH₂SI(OCH₃)₃, (CF₃)₂CF(CF₂)₈CH₂CH₂SI(OCH₃)₃, CF₃ (C₆H₄)C₂H₄SI(OCH₃)₃, CF₃ (CF₂) ₃(C₆H₄)C₂H₄SI(OCH₃)₃, CF₃ (CF₂)₅(C₆H₄)C₂H₄Si(OCH₃) ₃, CF₃ (CF₂)₇(C₆H₄)C₂H₄Si(OCH₃) ₃, CF₃ (CF₂)₃CH₂CH₂SiCH₃(OCH₃)₂, CF₃(CF₂)₅CH₂CH₂SiCH₃(OCH₃)₂, CF₃ (CF₂)₇CH₂CH₂SiCH₃(OCH₃)₂, CF₃(CF₂)₉CH₂CH₂SiCH₃(OCH₃)₂, (CF₃) ₂CF(CF₂)₄CH₂CH₂SiCH₃ (OCH₃)₂, (CF₃)₂CF(CF₂)₆CH₂CH₂SiCH₃ (OCH₃)₂, (CF₃) ₂CF(CF₂)₈CH₂CH₂SiCH₃ (OCH₃)₂, CF₃ (C₆H₄)C₂H₄SiCH₃ (OCH₃)₂, CF₃ (CF₂)₃ (C₆H₄)C₂H₄SiCH₃ (OCH₃)₂, CF₃ (CF₂)₅(C₆H₄)C₂H₄SiCH₃ (OCH₃)₂, CF₃ (CF₂)₇(C₆H₄)C₂H₄SiCH₃ (OCH₃)₂, CF₃ (CF₂)₃CH₂CH₂Si(OCH₂CH₃)₃, CF₃ (CF₂)₅CH₂CH₂Si(OCH₂CH₃) ₃, or CF₃ (CF₂)₇CH₂CH₂Si(OCH₂CH₃) onto the nonplanar solid substrate or parts thereof. More specifically, the nonplanar solid substrate having a water contact angle of 70° to 95° can be obtained by self-assembled molecule (SAM) coating octadecyldimethyl(3-trimethoxysilyl propyl)ammonium (TMSAC) or tridecafluorotetrahydrooctyltrimethoxysilane (FTEOS) onto a SiO₂ layer of the nonplanar solid substrate.

In the device for isolating and amplifying a nucleic acid according to the current embodiment of the present invention, the nonplanar solid substrate may have at least one amine-based functional group at its surface. The surface with the amine-based functional group may be obtained by coating polyethyleneiminetrimethoxysilane (PEIM), aminopropyltriethoxysilane, N-(3-trimethoxysily)-propyl)ethylenediamine, and/or N-trimethoxysilylpropy-N,N,N-chloride trimethylammonium onto the nonplanar solid substrate or parts thereof. For example, a SiO2 layer of a solid support may be coated with an SAM of PEIM. The amine-based functional group is positively charged at a pH of 3.0-6.0.

In the device for isolating and amplifying a nucleic acid according to the current embodiment of the present invention, the heating unit can be any device known in the art which can be used to heat and/or the chamber. The heating unit can be a heater or a micro heater. The heating unit may comprise a single device capable of heating and cooling the chamber or may comprise one device for heating and another device for cooling the chamber.

In the device for isolating and amplifying a nucleic acid according to the current embodiment of the present invention, the temperature controlling unit can be any controller known in the art which can control the heating unit to generate a temperature cycle used in PCR. The temperature-controlling unit may include a temperature sensor that senses the temperature of the chamber and a device that controls on/off operation of the heating unit.

The present invention will be described in further detail with reference to the following examples. These examples are for illustrative purposes only and are not intended to limit the scope of the present invention.

### Examples

### Example 1: Binding Properties of DNA to a Solid Substrate having a Pillar Structure

In the current example, the binding properties of DNA were investigated by introducing DNA samples having varying DNA concentrations into a fluidic device including an inlet, an outlet, and a chamber having a pillar array on a 10 mm × 23 mm substrate. In the pillar array, the distance between adjacent pillars was 12 µm, the height of each pillar was 100 µm, and the cross-sectional surface of each pillar was a regular square with sides of 25 µm.

In the pillar array, each pillar had a surface coated with PEIM, a material having at least one amine-based functional group; or a surface coated with TMSAC and having a water contact angle of 70° to 95°, more specifically, a water contact angle of 80°.

DNA in 0.01 N NaOH (pH 12) and DNA in 100 mM NaH₂PO₄(pH 7.0) were used as DNA samples, and 200 µℓ of each DNA sample was conveyed at a flow rate of 200 µℓ/minute.

The DNA bound to the pillar array in the fluidic device was measured using a Picogreen kit (obtained from Molecular Probes Inc) and a spectroscope according to the instructions provided by the manufacturer.

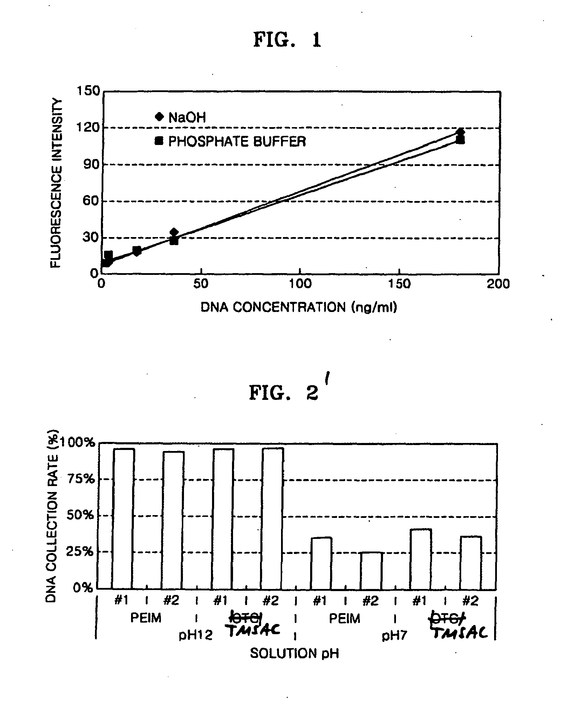

FIG. 1 is a graph showing the fluorescence intensity measured with respect to varying concentrations of DNA in a NaOH solution of pH 12.0 and in a phosphate buffer of pH 7.0. Referring to FIG. 1, the concentration of DNA is proportional to the fluorescence intensity, and thus, it was found that the concentration of DNA could be calculated using the proportional equation based on the relation between a fluorescence intensity and DNA concentration obtained.

FIG. 2 is a graph displaying the DNA elution rate with respect to the pH of the DNA sample. The DNA elution rate refers to the amount of DNA eluted from the fluidic device in relation to the amount of DNA in the sample that was introduced into the fluidic device with a pillar array. Referring to FIG. 2, when DNA was in NaOH of pH 12.0,95% or more of the initial DNA was recollected. On the other hand, when the DNA was contained in a phosphate buffer of pH 7.0, about 40% or less of DNA was recollected. Such results shows that, when the pH of the liquid environment for binding DNA to the solid substrate is high, such as in the range of 11 to 14, DNA was not efficiently bound to the solid substrate. But, when the pH is relatively low, such as in the range of 3 to 8, preferably 3 to 6, DNA was efficiently bound to the solid substrate. Accordingly, it was found that by using such a feature of DNA with respect to pH, DNA can be easily bound to or eluted from a solid substrate having a pillar structure. That is, binding of DNA to a solid substrate should be performed at a low pH, and eluting of DNA from the solid substrate should be performed at a high pH.

### Example 2: Binding of Cells to a Solid Substrate with a Pillar Array in a Fluidic Device, Cell Lysis, and DNA Elution

In the current example, a cell sample was introduced into a fluidic device including an inlet, an outlet, and a chamber having a pillar array formed on a 10 mm × 23 mm substrate, and then the cells were lysed to elute DNA. In the pillar array, the distance between adjacent pillars was 12 µm, the height of each pillar was 100 µm, and the cross-sectional surface of each pillar was a regular square having sides of 25 µm.

The pillar array had a surface coated with TMSAC having a water contact angle of 70° to 95°, more specifically, 80°.

The cell sample was an *E.coli* suspension of 0.01 OD₆₀₀ in a LB medium. 250 µℓ of the cell sample was loaded at a flow rate of 200 µℓ/minute.

Subsequently, *E.coli* that was bound to the surface of the pillar array was lysed. The cell lysis was performed by injecting 50 µℓ of 0.01 N NaOH solution at a flow rate of 25 µℓ/minute for 2 minutes (hereinafter referred to as 0.01 N NaOH_2), by injecting 50 µℓ of 0.01 N NaOH solution at a flow rate of 5 µℓ/minute for 10 minutes (hereinafter referred to as 0.01 N NaOH_10), or by injecting 3.5 µℓ of 0.01 N NaOH to the chamber, boiling the injected NaOH for 2 minutes, and then injecting 46.5 µℓ of 0.01 N NaOH solution at a flow rate of 200 µℓ/minute (hereinafter referred to as 0.01 N NaOH/boiling 3).

40 µℓ of the resulting lysate obtained by the cell lysis according to each of the above three exemplary methods was collected and the concentration of DNA therein was measured using a Picogreen kit (obtained from Molecular Probes Inc) and a spectroscope. The DNA elution efficiency was determined using the following formula: (fluorescence intensity of the sample/the expected fluorescence intensity of the cell when 100% of the cells are lysed)x 100. A DNA reference plot was obtained by lysing various samples containing *E.coli* having a known concentration using NaOH in a tube to obtain the fluorescence intensity with respect to the cell concentration.

The method of obtaining the expected fluorescence intensity of the cell when 100% of the cell was lysed will now be described. The amount of input cells = 10⁷ cells/ml (assuming 1.0 OD₆₀₀ as 10⁹ cell/ml) × 0.25 ml = 2.5×10⁶ cells, (assuming that the binding efficiency was 100%). Therefore, the expected maximum amount of eluted DNA = 2.5×10⁶ cells × 5 fg DNA / *E.coli* cell = 12.5 ng. Then, the amount of eluted DNA was divided by about 50 µℓ that was the entire volume of NaOH used as the eluting solution. As a result, the obtained concentration of DNA was 0.25 ng/µℓ. The expected fluorescence intensity, when 100% of cell was lysed, was obtained by inputting a DNA concentration of 0.25 ng/µℓ into the reference plot obtained.

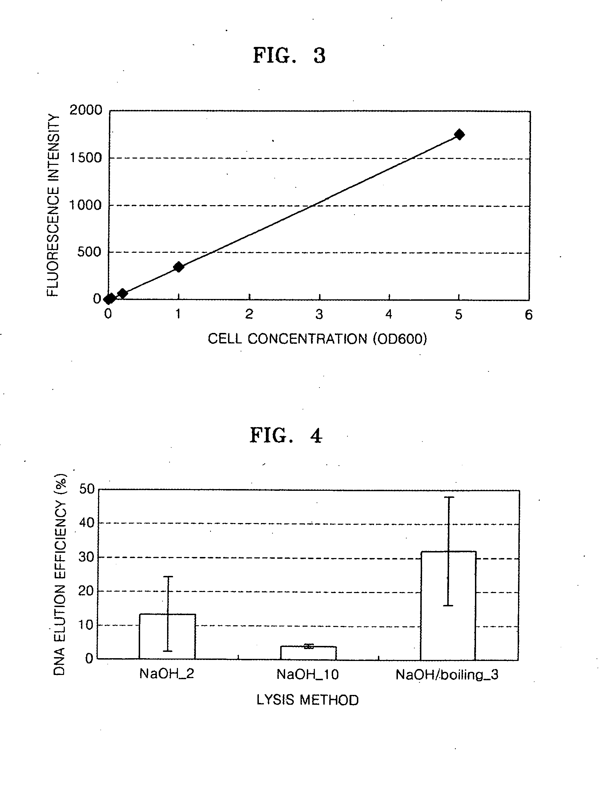

FIG. 3 is a graph of the cell concentration with respect to the fluorescence intensity measured in a cell lysate after cell lysis, which proportionally corresponds to the DNA concentration present in a cell lysate after cell lysis. Referring to FIG. 3, the cell concentration is proportional to the fluorescence intensity and respectively to the concentration of DNA in the cell lysate.

FIG. 4 is a graph illustrating the DNA elution efficiency determined according to the method by which the bound cells were lysed. As illustrated in FIG. 4, when NaOH was used, DNA elution efficiency was high when boiling of the NaOH solution was performed. When 0.01 N NaOH/boiling was performed, 32% of the DNA was collected assuming 1.0 OD₆₀₀ as 10⁹ cell/ml. As such, it was found that the DNA elution efficiency after the cell lysis was in the range of 30-60% in consideration of the distribution of cell concentration according to OD value (generally, 1.0 OD₆₀₀ is 5×10⁸ to 10⁹ *E.coli* cells/ml.)

### Example 3: Binding of Cells to a Solid Substrate with a Pillar Array in a Fluidic Device, Cell Lysis, and DNA Purification and Amplification

In the current example, a cell sample was introduced into a fluidic device including an inlet and an outlet and having a pillar array on a 7.5 mm × 15 mm substrate, the cells were lysed to bind DNA derived from the cell lysate to the substrate, matters that were not bound to the substrate were removed by washing, and DNA amplification was performed using the DNA bound to the substrate as a template. In the pillar array, the distance between adjacent pillars was 15 µm, the height of each pillar was 100 µm, and the cross-sectional surface of each pillar was a regular square having sides of 25 µm.

The pillar array had a surface coated with TMSAC having a water contact angle of 70° to 95°, more specifically, 80°.

The cell sample was an *E*.*coli*-containing sample suspension of 0.01 OD₆₀₀ in a LB medium. The *E.coli*-containing sample was adjusted to have a pH of 4.0 by using 100 mM sodium acetate buffer, and was conveyed from the inlet to the outlet through the chamber at a flow rate of 100 µℓ/minute for five minutes.

Then, *E.coli* cells bound to the surface of the pillar array were lysed. More specifically, the chamber was filled with 100 mM phosphate buffer (pH 4.0), treated at 95 °C for 2 minutes, and then the temperature was decreased to room temperature. Such lysis process was repeated five times. After the cell lysis, impurities which were not bound to the surface of the pillar array were washed using 200 µℓ of 100 mM phosphate buffer (pH 4.0) at a flow rate of 200 µℓ/minute.

Subsequently, PCR was performed using the DNA bound to the surface of the pillar array of the solid substrate as a template. The PCR was performed using a Taqman probe.

The qPCR was conducted using a Lightcycler^{™} 2.0 Real-Time PCR system from Roche Company, and the Ct value was calculated automatically by using the method stored in the Lightcycler^{™} 2.0 Real-Time PCR system.

Table 1 shows results of the real time PCR amplification after the *E.coli*-containing sample was flowed through the chamber having a pillar array of a fluidic device and underwent cell lysis and cell washing.

**Table 1**

| Sample No. | Treatment | Ct |
|---|---|---|
| 1 | dPCR1 | 14.14 |
| 2 | dPCR2 | 14.13 |
| 3 | Purified PCR1 | 13.29 |
| 4 | Purified PCR2 | 14.63 |
| 5 | 0.01 OD cell | 25.72 |
| 6 | Negative Control group | 25.23 |

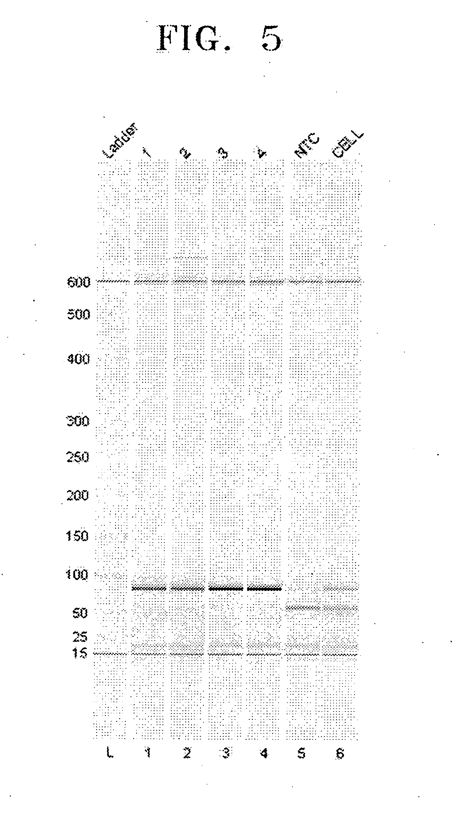

In Table 1, purified PCR refers to a PCR using lysed/washed sample (Lanes 3 and 4 of the electrophoresis of Fig. 5), and dPCR refers to a PCR using a sample that was not lysed and washed (Lanes of 1 and 2 of the electrophoresis of Fig.5). 0.01 OD cell (Lane 6 of the electrophoresis of Fig. 5) and negative control group (Lane 5 of the electrophoresis of Fig. 5) refer to a result for sample which does not include *E.coli* cell, respectively. As shown in Table 1, when the *E.coli*-containing sample was flowed through a chamber having a surface with a pillar array of a fluidic device, and underwent cell lysis, washing, and real time PCR amplification, 10 or less PCR cycles than a 0.01 OD cell reference sample was determined (DNA concentration rate: 1,000 times). Meanwhile, the purified PCR samples, in which the *E.coli* cells underwent binding, cell lysis, washing, and PCR, showed similar Ct values to the dPCR samples, in which the *E.coli* cells were bound and underwent PCR without cell lysis. Such results may result from high concentration and purification effect at a cell level. That is, it is assumed that the purification effect at the DNA level may be negligible.

FIG. 5 shows the electrophoresis results illustrating the concentration of amplified DNA measured according to a method in which the *E.coli*-containing sample was flowed through a chamber having a surface with a pillar array of a fluidic device, underwent cell lysis, cell washing, and real time PCR amplification, and electrophoresis. Referring to FIG. 5, Lanes 1 and 2 show the results obtained when the *E.coli* was bound and then underwent PCR without cell lysis, Lanes 3 and 4 shows results obtained when *E.coli* was bound and underwent cell lysis and PCR, and Lane 6 shows results obtained from PCR using 0.01 OD *E.coli*-containing sample as a template. The concentrations of the final products measured are shown in Table 2. The concentration of the final product was increased by 70% or more, when the cell lysis was included, even though samples with and without cell lysis showed similar Ct values.

**Table 2**

| | Purified PCR | dPCR | 0.01 OD cell |
|---|---|---|---|
| DNA concentration (ng/µℓ) | 24.45 | 17.35 | 7.5 |
| Increase rate compared to 0.01 OD cell | 226% | 131% | 1 |

### Example 4: Binding of Cell to a Solid Substrate with a Pillar Array in a Fluidic Device, Cell Lysis, and DNA Purification and Amplification, Using a Clinical Mimic Sample

In the current example, a urine sample containing *E-coli* cells was introduced into a fluidic device including an inlet and an outlet and having an pillar array on a 7.5 mm × 15 mm substrate, the cells were lysed and the DNA derived from the cell lysate was bound to the substrate, unbound materials that were not bound to the substrate were removed by washing, and the DNA was amplified using the DNA bound to the substrate as a template. In the pillar array, the distance between adjacent pillars was 15 µm, the height of each pillar was 100 µm, and the cross-sectional surface of each pillar was a regular square having sides of 25 µm.

The pillar array had a surface coated with TMSAC having a water contact angle of 70° to 95°, more specifically, 80°.

The cell sample was a urine sample which was diluted with a sodium acetate buffer (pH 4.0) in a ratio of 4:1. *E.coli* cells were added to the diluted sample, which diluted sample was of 0.01 OD₆₀₀. The *E.coli*-containing urine sample was conveyed from the inlet to the outlet through the chamber at a flow rate of 100 µℓ/minute for five minutes. As a reference sample, a 0.01 OD *E.coli*-containing sample (pH 4) in sodium acetate buffer was used.

Then, *E.coli* bound to the surface of the pillar array was lysed. More specifically, the chamber was filled with 100 mM phosphate buffer (pH 4.0), treated at 95 °C for 2 minutes, and then the temperature was decreased to room temperature. Such lysis process was repeated five times. After the cell lysis, impurities, which were not bound to the surface of the pillar array, were washed using 200 µℓ of 100 mM phosphate buffer (pH 4.0) at a flow rate of 200 µℓ/minute. Subsequently, PCR was performed using the DNA bound to the surface of the pillar array of the solid substrate as a template, using Sybr Green.

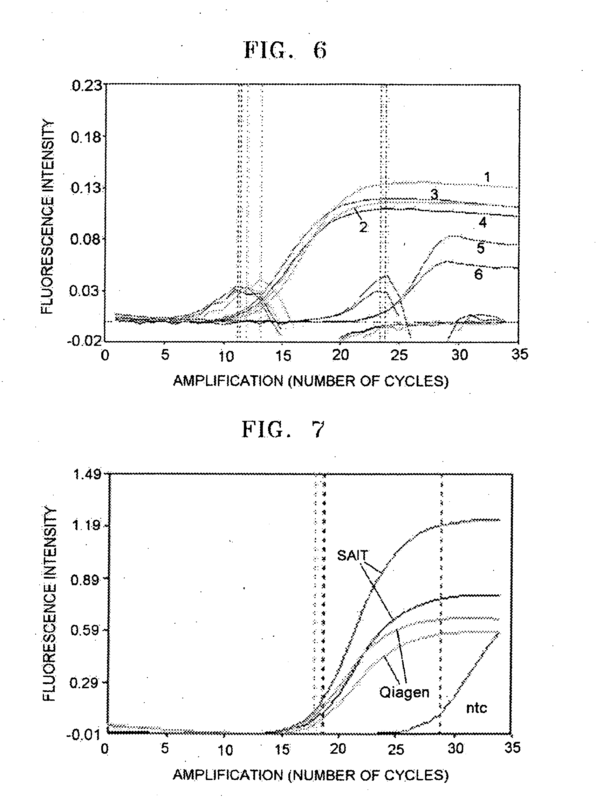

FIG. 6 is a graph illustrating the results of the real time PCR amplification after the *E.coli*-containing urine sample was flowed through the chamber having a pillar array of a fluidic device and underwent cell lysis and cell washing. Referring to FIG. 6, samples according to Table 3 were used.

**Table 3**

| Sample No. | Treatment | Ct(minute) |
|---|---|---|
| 1 | Purified PCR1 | 11.43 |
| 2 | Purified PCR2 | 11.21 |
| 3 | dPCR1 | 11.94 |
| 4 | dPCR 2 | 13.16 |
| 5 | Negative Control group 1 | 23.76 |
| 6 | Negative Control group 2 | 23.38 |

In Table 3, purified PCR refers to a PCR using lysed and washed sample and dPCR refers to a PCR using a sample that was not lysed and washed.

Referring to FIG. 6, the Ct value was about one cycle lower (1.23) when an *E.coli*-containing urine sample was flowed through a chamber having a surface with a pillar array of a fluidic device, and underwent cell lysis, cell washing, and real time PCR (purified PCR) than when the *E*.*coli*-containing urine sample was flowed through the chamber having a surface with a pillar array of a fluidic device, and underwent real time PCR without the cell lysis and washing (dPCR). In the Negative Control group 1 and 2, PCR was conducted using the E.*coli*-containing urine sample as template directly, without undergoing the purification process.

### Example 5: Binding of Cells to a Solid Substrate with a Pillar Array in a Fluidic Device, Cell Lysis, and DNA Elution and Amplification, Using a Clinical Mimic Sample - Comparative Example with respect to commercially available DNA purification kit

In the current example, an *E. coli*-containing whole blood sample was introduced into a fluidic device including a 10 mm × 23 mm substrate with a pillar array, and then underwent cell lysis to elute the DNA from the substrate. Then, the DNA was amplified using the eluted DNA as a template. This method was compared to a commercially available DNA purified kit produced by Qiagen Inc.

In the pillar array, the distance between adjacent pillars was 12 µm, the height of each pillar was 100 µm, and the cross-sectional surface of each pillar was a regular square having sides of 25 µm. The pillar array had a surface coated with TMSAC having a water contact angle of 70° to 95°, more specifically, 80°.

The clinical mimic cell sample containing *E.coli* cells and was prepared by adding 10 µℓ of an *E.coli* suspension of 1.0 OD₆₀₀ to 1 ml of whole blood. 200 µℓ of the clinical mimic cell sample containing *E.coli* cells was diluted with sodium acetate buffer (pH 3.0, 100mM) 1:1 and then the diluted sample was conveyed from an inlet to an outlet through the chamber at a flow rate of 200 µℓ/minute. The *E.coli* cells bound to the surface of the pillar array were lysed. In order to perform the cell lysis, 5 µℓ of 0.01 N NaOH was added to the chamber and then boiled for 2 minutes, and subsequently 45 µℓ of NaOH was added to the chamber at a flow rate of 200 µℓ/min to elute DNA.

As a control sample (Qiagen Inc.'s kit, *Cat 13323, Blood & Cell Culture DNA Mini* Kit), 200 µℓ of the clinical mimic sample was prepared according to a protocol provided by Qiagen Inc.

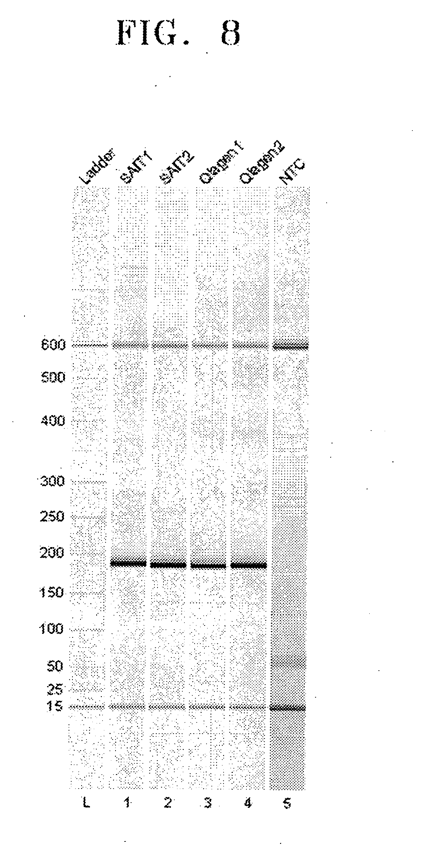

Then, the clinical mimic cell sample containing *E.coli* cells and the control group were subjected to PCR (Sybr Green). As shown in Table 4, the two methods showed similar Ct values and similar PCR product concentrations. That is, it was found that the quality and quantity of DNA prepared according to the method of the present invention was high enough to undergo PCR, not requiring additional DNA purification. These methods, however, required different times to prepare DNA. The method of the present invention required about 10 minutes, and the Qiagen method required about 45 minutes. In addition, the method of the present invention can be easily automated because cell capturing and DNA elution can be performed on a single chip. Therefore, the method of the present invention is very useful to manufacture a LOC. In Table 4, SAIT represents the results of the method of the present invention, and the concentration represents the results of electrophoresis illustrated in FIG. 8. In Table 4, in the Negative Control group, PCR was conducted using the E. coli-containing whole blood sample as a template, without undergoing a purification process.

**Table 4**

| Chip | CT | Concentration (ng/µℓ) |
|---|---|---|
| SAIT1 | 18.7 | 11.8 |
| SAIT2 | 18.9 | 13.1 |
| Qiagen1 | 18.4 | 13.0 |
| Qiagen2 | 17.8 | 11.5 |
| Negative Control Group | 28.8 | - |

FIG. 7 is a graph illustrating the results of the real time PCR amplification after an *E.coli*-containing whole blood sample was flowed through a chamber having a pillar array of a fluidic device and underwent cell lysis and DNA elution.

FIG. 8 is a graph illustrating the results of electrophoresis after an *E.coli*-containing whole blood sample was flowed through a chamber having a pillar array of a fluidic device and underwent cell lysis, DNA elution, and real time PCR amplification.

In the method of isolating a nucleic acid from a microorganism cell according to the present invention, isolation of a microorganism cell and isolation of a nucleic acid can be efficiently performed at the same time, i.e. straight forward in a single container. In addition, a nucleic acid separator, such as a chaotropic substance, is not required for isolation of the nucleic acid. Furthermore, the method can be usefully realized using a small device, such as lap-on-a-chip (LOC).

In the method of amplifying DNA according to the present invention, isolation of a cell and isolation of a nucleic acid derived from the cell can be continually performed so that the nucleic acid can be effectively amplified using a small device, such as a LOC.

By using a device for isolating and amplifying a nucleic acid according to the present invention, isolation of a microorganism cell, isolation of a nucleic acid derived from the microorganism cell, and amplification of the nucleic acid using the nucleic acid derived from the microorganism cell as a template can be performed in the same container or in different containers.

While the present invention has been particularly shown and described with reference to exemplary embodiments thereof, it will be understood by those of ordinary skill in the art that various changes in form and details may be made therein without departing from the scope of the present invention as defined by the following claims.

## Claims

1. A method of isolating a nucleic acid from a cell, the method comprising:
contacting a nonplanar solid substrate with a cell-containing sample in a liquid medium having a pH of 3.0 to 6.0 and binding cells to the nonplanar solid substrate; and
lysing the cells bound to the nonplanar solid substrate and isolating a nucleic acid from the cell lysate,
wherein the cell is a microorganism cell, wherein the nonplanar solid substrate has a surface having a water contact angle of 70° to 95°, wherein the nonplanar solid substrate is selected from the group consisting of a solid substrate having a surface comprising a pillar structure formed of a plurality of pillars, a bead-shaped solid substrate, and a sieve-shaped solid substrate having a surface comprising pores, and wherein a surface of the nonplanar solid substrate is coated with octadecyldimethyl(3-trimethoxysilyl propyl)ammonium (TMSAC) or tridecafluorotetrahydrooctyltrimethoxysilane (FTEOS) or polyethyleneiminetrimethoxysilane (PEIM).

2. The method according to claim 1, wherein the cell is bacteria, fungus, or a virus.

3. The method according to claim 1 or 2, wherein the cell-containing sample is a biological sample.

4. The method according to claim 3, wherein the biological sample is blood, urine, or saliva.

5. The method according to any of claims 1 to 4, wherein the cell-containing sample is diluted in the liquid medium, which is a phosphate buffer or an acetate buffer.

6. The method according to claim 5, wherein the cell-containing sample is diluted in a ratio of 1:1 to 1:10.

7. The method according to any of claims 1 to 6, wherein the liquid medium has a salt concentration of 10 mM to 500 mM.

8. The method according to claim 7, wherein the liquid medium has a salt concentration of 50 mM to 300 mM.

9. The method according to claim 1, wherein the nonplanar solid substrate comprises a solid substrate having a surface comprising a pillar structure, wherein each of the pillars has an aspect ratio of 1:1 to 20:1, wherein the aspect ratio is height of a pillar : the length of the cross-section.

10. The method according to claim 1 or 9, wherein the nonplanar solid substrate comprises a solid substrate having a surface comprising a pillar structure, wherein the ratio of the height of the pillars to the distance between adjacent pillars is in the range of 1:1 to 25:1.

11. The method according to any of claims 1, 9 or 10, wherein the nonplanar solid substrate comprises a solid substrate having a surface comprising a pillar structure, wherein the distance between adjacent pillars is in the range of 5 µm to 100 µm.

12. The method according to any of claims 1 to 11, further comprising, after the contacting step and prior to the lysing step, washing the nonplanar solid substrate to remove unbound matters, which are not bound to the nonplanar solid substrate.

13. The method according to any of claims 1 to 12, wherein lysing the cells is performed by boiling lysis, laser lysis, lysis using a chemical compound, or electrochemical lysis.

14. The method according to any of claims 1 to 13, wherein the cells are lysed in a liquid medium having a pH of 3.0 to 6.0 for binding a nucleic acid derived from the cell lysate to the nonplanar solid substrate.

15. The method according to claim 14, further comprising, after the lysing step, washing the nonplanar solid substrate to remove unbound matters, which are not bound to the nonplanar solid substrate.

16. The method according to claim 14 or 15, further comprising eluting the nucleic acid bound to the nonplanar solid substrate.

17. The method according to any of claims 1 to 13, wherein the cells are lysed in a liquid medium having a pH of 11 to 14.

18. The method according to claim 17, further comprising extracting a nucleic acid from the cell lysate obtained.

19. A method of amplifying a target nucleic acid, the method comprising:
isolating a nucleic acid from a cell according to a method of any one of claims 1 to 18; and
amplifying the target nucleic acid using the isolated nucleic acid as a template.

20. A method of amplifying a target nucleic acid, the method comprising:
contacting a nonplanar solid substrate with a cell-containing sample in a liquid medium having a pH of 3.0 to 6.0 and binding cells to the nonplanar solid substrate, wherein the nonplanar solid substrate has a surface having a water contact angle of 70° to 95°, wherein the nonplanar solid substrate is selected from the group consisting of a solid substrate having a surface comprising a pillar structure formed of a plurality of pillars, a bead-shaped solid substrate, and a sieve-shaped solid substrate having a surface comprising pores, and wherein a surface of the nonplanar solid substrate is coated with octadecyldimethyl(3-trimethoxysilyl propyl)ammonium (TMSAC) or tridecafluorotetrahydrooctyltrimethoxysilane (FTEOS) or polyethyleneiminetrimethoxysilane (PEIM); and
lysing the cells bound to the nonplanar solid substrate, wherein the cell is a microorganism cell, and
amplifying the target nucleic acid using a nucleic acid directly present in the cell lysate as a template.

21. The method according to claim 19 or 20, wherein the nucleic acid is DNA or RNA.

22. The method according to any of claims 19 to 21, wherein amplifying the target nucleic acid is performed in a first chamber comprising the nonplanar solid substrate or in a second chamber, which is in fluid communication with the first chamber, after transferring the isolated nucleic acid or the cell lysate from the first chamber to the second chamber.

23. The method according to any of claims 19 to 22, wherein amplifying is performed by PCR.

24. A device for the isolation and amplification of a nucleic acid, the device comprising:
a reaction chamber comprising a nonplanar solid substrate;
a heating unit for heating the reaction chamber; and
a temperature-controlling unit for controlling the heating unit, wherein the nonplanar solid substrate has a surface having a water contact angle of 70° to 95°, wherein the nonplanar solid substrate is selected from the group consisting of a solid substrate having a surface comprising a pillar structure formed of a plurality of pillars, a bead-shaped solid substrate, and a sieve-shaped solid substrate having a surface comprising pores, and wherein a surface of the nonplanar solid substrate is coated with octadecyldimethyl(3-trimethoxysilyl propyl)ammonium (TMSAC) or tridecafluorotetrahydrooctyltrimethoxysilane (FTEOS) or polyethyleneiminetrimethoxysilane (PEIM).

25. The device according to claim 24, further comprising a nucleic acid amplification chamber, which is in fluid communication with the reaction chamber comprising the nonplanar solid substrate.

26. The device according to claim 24, wherein the nonplanar solid substrate comprises a solid substrate having a surface comprising a pillar structure, wherein each of the pillars has an aspect ratio of 1:1 to 20:1, wherein the aspect ratio is height of a pillar : the length of the cross-section.

27. The device according to claim 24 or 26, wherein the nonplanar solid substrate comprises a solid substrate having a surface comprising a pillar structure, wherein the ratio of the height of the pillars to the distance between adjacent pillars is in the range of 1:1 to 25:1.

28. The device according to any of claims 24, 26 or 27, wherein the nonplanar solid substrate comprises a solid substrate having a surface comprising a pillar structure, wherein the distance between adjacent pillars is in the range of 5 µm to 100 µm.

## Patentansprüche

1. Verfahren zum Isolieren einer Nucleinsäure aus einer Zelle, wobei das Verfahren umfasst:
das Inkontaktbringen eines nicht-planaren festen Substrats mit einer zellhaltigen Probe in einem flüssigem Medium mit einem pH von 3,0 bis 6,0 und das Binden von Zellen an das nicht-planare feste Substrat; und
das Lysieren der an das nicht-planare feste Substrat gebundenen Zellen und das Isolieren einer Nucleinsäure aus dem Zelllysat,
wobei die Zelle eine Mikroorganismenzelle ist, wobei das nicht-planare feste Substrat eine Oberfläche mit einem Wasserkontaktwinkel von 70° bis 95° aufweist, wobei das nicht-planare feste Substrat ausgewählt ist aus der Gruppe bestehend aus einem festen Substrat mit einer Oberfläche, die eine aus einer Mehrzahl von Säulen gebildete Säulenstruktur umfasst, einem perlenförmigen festen Substrat und einem siebförmigen festen Substrat mit einer Oberfläche, die Poren umfasst, und wobei eine Oberfläche des nicht-planaren festen Substrats mit Octadecyldimethyl(3-trimethoxysilylpropyl)ammonium (TMSAC) oder Tridecafluortetrahydrooctyltrimethoxysilan (FTEOS) oder Polyethylenimintrimethoxysilan (PEIM) beschichtet ist.

2. Verfahren nach Anspruch 1, wobei es sich bei der Zelle um ein Bakterium, einen Pilz oder ein Virus handelt.

3. Verfahren nach Anspruch 1 oder 2, wobei die zellhaltige Probe eine biologische Probe ist.

4. Verfahren nach Anspruch 3, wobei es sich bei der biologischen Probe um Blut, Urin oder Speichel handelt.

5. Verfahren nach einem der Ansprüche 1 bis 4, wobei die zellhaltige Probe in dem flüssigen Medium verdünnt wird, welches ein Phosphatpuffer oder ein Acetatpuffer ist.

6. Verfahren nach Anspruch 5, wobei die zellhaltige Probe in einem Verhältnis von 1:1 bis 1:10 verdünnt wird.

7. Verfahren nach einem der Ansprüche 1 bis 6, wobei das flüssige Medium eine Salzkonzentration von 10 mM bis 500 mM aufweist.

8. Verfahren nach Anspruch 7, wobei das flüssige Medium eine Salzkonzentration von 50 mM bis 300 mM aufweist.

9. Verfahren nach Anspruch 1, wobei das nicht-planare feste Substrat ein festes Substrat mit einer Oberfläche umfasst, die eine Säulenstruktur umfasst, wobei jede der Säulen ein Aspektverhältnis von 1:1 bis 20:1 aufweist, wobei das Aspektverhältnis die Höhe einer Säule : die Länge des Querschnitts ist.

10. Verfahren nach Anspruch 1 oder 9, wobei das nicht-planare feste Substrat ein festes Substrat mit einer Oberfläche umfasst, die eine Säulenstruktur umfasst, wobei das Verhältnis der Höhe der Säulen zu dem Abstand zwischen benachbarten Säulen im Bereich von 1:1 bis 25:1 liegt.

11. Verfahren nach einem der Ansprüche 1, 9 oder 10, wobei das nicht-planare feste Substrat ein festes Substrat mit einer Oberfläche umfasst, die eine Säulenstruktur umfasst, wobei der Abstand zwischen benachbarten Säulen im Bereich von 5 µm bis 100 µm liegt.

12. Verfahren nach einem der Ansprüche 1 bis 11, außerdem umfassend, nach dem Schritt des Inkontaktbringens und vor dem Schritt des Lysierens, das Waschen des nicht-planaren festen Substrats, um ungebundene Substanzen zu entfernen, welche nicht an das nicht-planare feste Substrat gebunden sind.

13. Verfahren nach einem der Ansprüche 1 bis 12, wobei das Lysieren der Zellen mittels Lyse durch Sieden, Laserlyse, Lyse unter Verwendung einer chemischen Verbindung oder elektrochemische Lyse durchgeführt wird.

14. Verfahren nach einem der Ansprüche 1 bis 13, wobei die Zellen in einem flüssigen Medium mit einem pH von 3,0 bis 6,0 lysiert werden, um eine aus dem Zelllysat gewonnene Nucleinsäure an das nicht-planare feste Substrat zu binden.

15. Verfahren nach Anspruch 14, außerdem umfassend, nach dem Scritt des Lysierens, das Waschen des nicht-planaren festen Substrats, um ungebundene Substanzen zu entfernen, welche nicht an das nicht-planare feste Substrat gebunden sind.

16. Verfahren nach Anspruch 14 oder 15, außerdem umfassend das Eluieren der an das nicht-planare feste Substrat gebundenen Nucleinsäure.

17. Verfahren nach einem der Ansprüche 1 bis 13, wobei die Zellen in einem flüssigen Medium mit einem pH von 11 bis 14 lysiert werden.

18. Verfahren nach Anspruch 17, außerdem umfassend das Extrahieren einer Nucleinsäure aus dem erhaltenen Zelllysat.

19. Verfahren zum Amplifizieren einer Zielnucleinsäure, wobei das Verfahren umfasst:
das Isolieren einer Nucleinsäure aus einer Zelle gemäß einem Verfahren nach einem der Ansprüche 1 bis 18; und
das Amplifizieren der Zielnucleinsäure unter Verwendung der isolierten Nucleinsäure als eine Matrize.

20. Verfahren zum Amplifizieren einer Zielnucleinsäure, wobei das Verfahren umfasst:
das Inkontaktbringen eines nicht-planaren festen Substrats mit einer zellhaltigen Probe in einem flüssigem Medium mit einem pH von 3,0 bis 6,0 und das Binden von Zellen an das nicht-planare feste Substrat, wobei das nicht-planare feste Substrat eine Oberfläche mit einem Wasserkontaktwinkel von 70° bis 95° aufweist, wobei das nicht-planare feste Substrat ausgewählt ist aus der Gruppe bestehend aus einem festen Substrat mit einer Oberfläche, die eine aus einer Mehrzahl von Säulen gebildete Säulenstruktur umfasst, einem perlenförmigen festen Substrat und einem siebförmigen festen Substrat mit einer Oberfläche, die Poren umfasst, und wobei eine Oberfläche des nicht-planaren festen Substrats mit Octadecyldimethyl(3-trimethoxysilylpropyl)ammonium (TMSAC) oder Tridecafluortetrahydrooctyltrimethoxysilan (FTEOS) oder Polyethylenimintrimethoxysilan (PEIM) beschichtet ist; und
das Lysieren der an das nicht-planare feste Substrat gebundenen Zellen, wobei die Zelle eine Mikroorganismenzelle ist, und
das Amplifizieren der Zielnucleinsäure unter Verwendung einer Nucleinsäure, die in dem Zelllysat direkt vorhanden ist, als Matrize.

21. Verfahren nach Anspruch 19 oder 20, wobei es sich bei der Nucleinsäure um DNA oder RNA handelt.

22. Verfahren nach einem der Ansprüche 19 bis 21, wobei das Amplifizieren der Zielnucleinsäure in einer ersten Kammer, die das nicht-planare feste Substrat umfasst, oder in einer zweiten Kammer, welche in Flüssigkeitsverbindung mit der ersten Kammer steht, nach dem Überführen der isolierten Nucleinsäure oder des Zelllysats aus der ersten Kammer in die zweite Kammer durchgeführt wird.

23. Verfahren nach einem der Ansprüche 19 bis 22, wobei das Amplifizieren mittels PCR durchgeführt wird.

24. Vorrichtung für die Isolierung und Amplifikation einer Nucleinsäure, wobei die Vorrichtung umfasst:
eine Reaktionskammer, die ein nicht-planares festes Substrat umfasst;
ein Heizelement zum Erwärmen der Reaktionskammer; und
eine Temperaturregelungseinheit zum Regeln des Heizelements, wobei das nicht-planare feste Substrat eine Oberfläche mit einem Wasserkontaktwinkel von 70° bis 95° aufweist, wobei das nicht-planare feste Substrat ausgewählt ist aus der Gruppe bestehend aus einem festen Substrat mit einer Oberfläche, die eine aus einer Mehrzahl von Säulen gebildete Säulenstruktur umfasst, einem perlenförmigen festen Substrat und einem siebförmigen festen Substrat mit einer Oberfläche, die Poren umfasst, und wobei eine Oberfläche des nicht-planaren festen Substrats mit Octadecyldimethyl(3-tri-methoxysilylpropyl)ammonium (TMSAC) oder Tridecafluortetrahydrooctyltrimethoxysilan (FTEOS) oder Polyethylenimintrimethoxysilan (PEIM) beschichtet ist.

25. Vorrichtung nach Anspruch 24, außerdem umfassend eine Nucleinsäureamplifikationskammer, welche in Flüssigkeitsverbindung mit der Reaktionskammer steht, die das nicht-planare feste Substrat umfasst.

26. Vorrichtung nach Anspruch 24, wobei das nicht-planare feste Substrat ein festes Substrat mit einer Oberfläche umfasst, die eine Säulenstruktur umfasst, wobei jede der Säulen ein Aspektverhältnis von 1:1 bis 20:1 aufweist, wobei das Aspektverhältnis die Höhe einer Säule : die Länge des Querschnitts ist.

27. Vorrichtung nach Anspruch 24 oder 26, wobei das nicht-planare feste Substrat ein festes Substrat mit einer Oberfläche umfasst, die eine Säulenstruktur umfasst, wobei das Verhältnis der Höhe der Säulen zu dem Abstand zwischen benachbarten Säulen im Bereich von 1:1 bis 25:1 liegt.

28. Vorrichtung nach einem der Ansprüche 24, 26 oder 27, wobei das nicht-planare feste Substrat ein festes Substrat mit einer Oberfläche umfasst, die eine Säulenstruktur umfasst, wobei der Abstand zwischen benachbarten Säulen im Bereich von 5 µm bis 100 µm liegt.

## Revendications

1. Procédé d'isolement d'un acide nucléique à partir d'une cellule, le procédé comprenant :
la mise en contact d'un substrat solide non plan avec un échantillon contenant des cellules dans un milieu liquide ayant un pH de 3,0 à 6,0 et la liaison des cellules au substrat solide non plan; et
la lyse des cellules liées au substrat solide non plan et l'isolement d'un acide nucléique à partir du lysat cellulaire,
dans lequel la cellule est une cellule de micro-organisme, dans lequel le substrat solide non plan a une surface ayant un angle de contact avec l'eau de 70° à 95°, dans lequel le substrat solide non plan est choisi dans le groupe constitué d'un substrat solide ayant une surface comprenant une structure de type pilier formée d'une pluralité de piliers, d'un substrat solide en forme de bille, et d'un substrat solide en forme de tamis ayant une surface comprenant des pores, et dans lequel une surface du substrat solide non plan est revêtue d'octadécyldiméthyl(3-triméthoxysilylpropyl)ammonium (TMSAC) ou de tridécafluorotétrahydrooctyltriméthoxysilane (FTEOS) ou de polyéthylèneiminetriméthoxysilane (PEIM).

2. Procédé selon la revendication 1, dans lequel la cellule est une bactérie, un champignon, ou un virus.

3. Procédé selon la revendication 1 ou 2, dans lequel l'échantillon contenant des cellules est un échantillon biologique.

4. Procédé selon la revendication 3, dans lequel l'échantillon biologique est du sang, de l'urine ou de la salive.

5. Procédé selon l'une quelconque des revendications 1 à 4, dans lequel l'échantillon contenant des cellules est dilué dans le milieu liquide, qui est un tampon phosphate ou un tampon acétate.

6. Procédé selon la revendication 5, dans lequel l'échantillon contenant des cellules est dilué dans un rapport de 1:1 à 1:10.

7. Procédé selon l'une quelconque des revendications 1 à 6, dans lequel le milieu liquide a une concentration en sel de 10 mM à 500 mM.

8. Procédé selon la revendication 7, dans lequel le milieu liquide a une concentration en sel de 50 mM à 300 mM.

9. Procédé selon la revendication 1, dans lequel le substrat solide non plan comprend un substrat solide ayant une surface comprenant une structure de type pilier, où chacun des piliers a un rapport de forme de 1:1 à 20:1, le rapport de forme étant la hauteur d'un pilier : la longueur de la section transversale.

10. Procédé selon la revendication 1 ou 9, dans lequel le substrat solide non plan comprend un substrat solide ayant une surface comprenant une structure de type pilier, où le rapport de la hauteur des piliers à la distance entre des piliers adjacents est compris entre 1:1 et 25:1.

11. Procédé selon l'une quelconque des revendications 1, 9 ou 10, dans lequel le substrat solide non plan comprend un substrat solide ayant une surface comprenant une structure de type pilier, où la distance entre des piliers adjacents se situe dans la plage de 5 µm à 100 µm.

12. Procédé selon l'une quelconque des revendications 1 à 11, comprenant en outre, après l'étape de mise en contact et avant l'étape de lyse, le lavage du substrat solide non plan pour éliminer les matières non liées, qui ne sont pas liées au substrat solide non plan.

13. Procédé selon l'une quelconque des revendications 1 à 12, dans lequel la lyse des cellules est effectuée par une lyse par ébullition, lyse par laser, lyse à l'aide d'un composé chimique, ou lyse électrochimique.

14. Procédé selon l'une quelconque des revendications 1 à 13, dans lequel les cellules sont lysées dans un milieu liquide ayant un pH de 3,0 à 6,0 pour la liaison d'un acide nucléique dérivé du lysat cellulaire au substrat solide non plan.

15. Procédé selon la revendication 14, comprenant en outre, après l'étape de lyse, le lavage du substrat solide non plan pour éliminer les matières non liées, qui ne sont pas liées au substrat solide non plan.

16. Procédé selon la revendication 14 ou 15, comprenant en outre l'élution de l'acide nucléique lié au substrat solide non plan.

17. Procédé selon l'une quelconque des revendications 1 à 13, dans lequel les cellules sont lysées dans un milieu liquide ayant un pH de 11 à 14.

18. Procédé selon la revendication 17, comprenant en outre l'extraction d'un acide nucléique à partir du lysat cellulaire obtenu.

19. Procédé d'amplification d'un acide nucléique cible, le procédé comprenant :
l'isolement d'un acide nucléique à partir d'une cellule conformément à un procédé selon l'une quelconque des revendications 1 à 18 ; et
l'amplification de l'acide nucléique cible à l'aide de l'acide nucléique isolé servant de matrice.

20. Procédé d'amplification d'un acide nucléique cible, le procédé comprenant :
la mise en contact d'un substrat solide non plan avec un échantillon contenant des cellules dans un milieu liquide ayant un pH de 3,0 à 6,0 et la liaison des cellules au substrat solide non plan, dans lequel le substrat solide non plan a une surface ayant un angle de contact avec l'eau de 70° à 95°, dans lequel le substrat solide non plan est choisi dans le groupe constitué d'un substrat solide ayant une surface comprenant une structure de type pilier formée d'une pluralité de piliers, d'un substrat solide en forme de bille, et d'un substrat solide en forme de tamis ayant une surface comprenant des pores, et dans lequel une surface du substrat solide non plan est revêtue d'octadécyldiméthyl(3-triméthoxysilylpropyl)ammonium (TMSAC) ou de tridécafluorotétrahydrooctyltriméthoxysilane (FTEOS) ou de polyéthylèneiminetriméthoxysilane (PEIM) ; et
la lyse des cellules liées au substrat solide non plan, la cellule étant une cellule de micro-organisme, et
l'amplification de l'acide nucléique cible à l'aide d'un acide nucléique directement présent dans le lysat cellulaire et servant de matrice.

21. Procédé selon la revendication 19 ou 20, dans lequel l'acide nucléique est de l'ADN ou de l'ARN.

22. Procédé selon l'une quelconque des revendications 19 à 21, dans lequel l'amplification de l'acide nucléique cible est effectuée dans une première chambre comprenant le substrat solide non plan ou dans une seconde chambre, qui est en communication fluidique avec la première chambre, après le transfert de l'acide nucléique isolé ou du lysat cellulaire de la première chambre vers la seconde chambre.

23. Procédé selon l'une quelconque des revendications 19 à 22, dans lequel l'amplification est effectuée par PCR.

24. Dispositif pour l'isolement et l'amplification d'un acide nucléique, le dispositif comprenant :
une chambre de réaction comprenant un substrat solide non plan ;
une unité de chauffage pour chauffer la chambre de réaction ; et
une unité de régulation de la température pour réguler l'unité de chauffage, dans lequel le substrat solide non plan a une surface ayant un angle de contact avec l'eau de 70° à 95°, dans lequel le substrat solide non plan est choisi dans le groupe constitué d'un substrat solide ayant une surface comprenant une structure de type pilier formée d'une pluralité de piliers, d'un substrat solide en forme de bille, et d'un substrat solide en forme de tamis ayant une surface comprenant des pores, et dans lequel une surface du substrat solide non plan est revêtue d'octadécyldiméthyl(3-triméthoxysilylpropyl)ammonium (TMSAC) ou de tridécafluorotétrahydrooctyltriméthoxysilane (FTEOS) ou de polyéthylèneiminetriméthoxysilane (PEIM).

25. Dispositif selon la revendication 24, comprenant en outre une chambre d'amplification d'acide nucléique, qui est en communication fluidique avec la chambre de réaction comprenant le substrat solide non plan.

26. Dispositif selon la revendication 24, dans lequel le substrat solide non plan comprend un substrat solide ayant une surface comprenant une structure de type pilier, où chacun des piliers a un rapport de forme de 1:1 à 20:1, le rapport de forme étant la hauteur d'un pilier : la longueur de la section transversale

27. Dispositif selon la revendication 24 ou 26, dans lequel le substrat solide non plan comprend un substrat solide ayant une surface comprenant une structure de type pilier, où le rapport de la hauteur des piliers à la distance entre des piliers adjacents est compris entre 1:1 et 25:1.

28. Dispositif selon l'une quelconque des revendications 24, 26 ou 27, dans lequel le substrat solide non plan comprend un substrat solide ayant une surface comprenant une structure de type pilier, où la distance entre des piliers adjacents se situe dans la plage de 5 µm à 100 µm.
